# EUROPEAN PATENT SPECIFICATION

(11) **EP 2 097 383 B1**
(45) Date of publication and mention of the grant of the patent: **08.02.2012**
(21) Application number: 07870531.6
(22) Date of filing: 30.11.2007
(51) Int. Cl.: C07D 223/16, C07C 217/56

(54) **Process for preparation of ivabradine hydrochloride**
Verfahren zur Herstellung von Ivabradinhydrochlorid
Procédé de préparation d'hydrochlorure d'ivabradine

(30) Priority: 30.11.2006 IN MU19662006
(43) Date of publication of application: 09.09.2009
(60) Divisional application: 11195861.7
(73) Proprietor: Cadila Healthcare Limited, Ahmedabad 380 015 Gujarat (IN)
(72) Inventor: DWIVEDI, Shriprakash Dhar, Ahmedabad 380 015 Gujarat (IN); KUMAR, Rajiv, Ahmedabad 380 015 Gujarat (IN); PATEL, Sunil Tribhovandas, Ahmedabad 380 015 Gujarat (IN); SHAH, Alpeshkumar Parvin Chandra, Ahmedabad 380 015 Gujarat (IN)
(74) Representative: Clarke, Lionel Paul
(86) International application number: PCT/IN2007/000564
(87) International publication number: WO 2008/065681

(56) References cited:
- EP-A- 0 534 859
- EP-A- 1 589 005
- EP-A- 1 598 333
- US-A- 4 490 369

## Description

The present invention provides a process for preparation of Ivabradine hydrochloride of formula (I), its pharmaceutically acceptable salts, solvates and hydrates thereof. Ivabradine is chemically known as 3-{3-[{[(7S)-3,4-dimethoxybicyclo[4.2.0]octa-1,3,5-trien-7-yl]methyl)(methyl)amino]-propyl)-7,8-dimethoxy-1,3,4,5-tet-ahydro-2H-3-benzazepin-2-one and its hydrochloride salt is useful in the treatment of myocardial ischaemia.

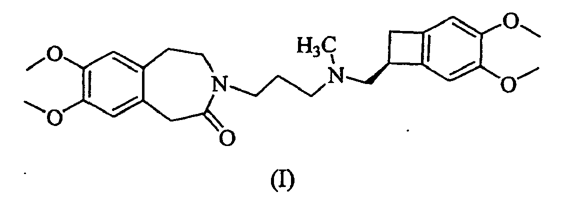

The present invention also relates to the novel intermediate of formula (IV) for the preparation of Ivabradine or its pharmaceutically acceptable salt, solvates thereof.

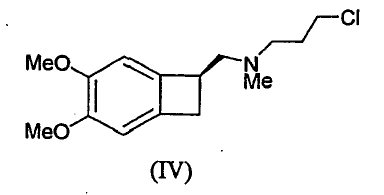

The present invention provides a novel intermediate for the preparation of Ivabradine hydrochloride with high yield and chiral purity.

### BACKGROUND OF THE INVENTION

Ivabradine hydrochloride, 3-{3-[([(7S)-3,4-dimethoxybicyclo[4.2.0]octa-1,3,5-trien-7-yl]methyl}(methyl)amino]-propyl}-7,8-dimethoxy-1,3,4,5-tetrahydro-2H-3-benzazepin-2-one hydrochloride of formula (Ia) have very valuable pharmacological and therapeutic properties, especially bradycardic properties, making those compounds useful in the treatment or prevention of various clinical situations of myocardial ischaemia such as angina pectoris, myocardial infarct and associated rhythm disturbances, and also of various pathologies involving rhythm disturbances, especially supraventricular rhythm disturbances, and in the treatment of heart failure.

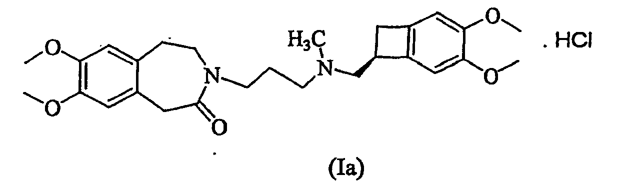

The preparation and therapeutic use of Ivabradine and salts thereof with a pharmaceutically acceptable acid, and more especially its hydrochloride, have been described in European Patent EP 0534859. This patent describes synthesis of Ivabradine hydrochloride by reacting the compound of formula (a) with the compound of formula (b): 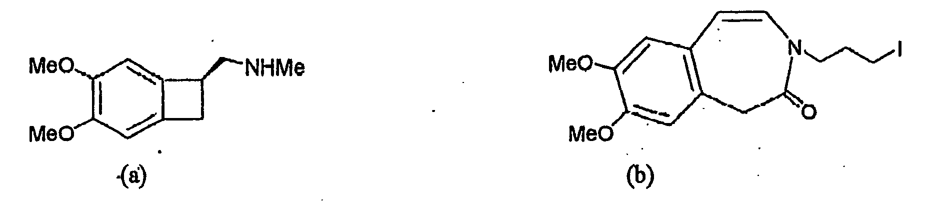 to give compound of formula (c), subsequent catalytic hydrogenation results in Ivabradine, which is further converted to its hydrochloride salt:

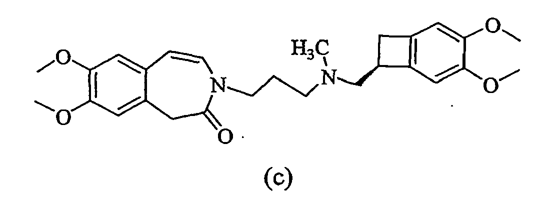

This disclosed process yields Ivabradine hydrochloride in a very low yield - less than 17% over the 3 steps as a whole.

Another process for preparing Ivabradine is disclosed in US 5296482. According to the process, (+) isomer of Ivabradine is treated with aqueous HCl and then recrystallization in acetonitrile leads to the formation of Ivabradine hydrochloride salt having M.P. 135-140°C.

US patent no. 7,176,197 B2 discloses a process for preparation of α - crystalline form of Ivabradine hydrochloride which comprises reacting 3-[2-(1,3-dioxolan-2-yl) ethyl]-7,8-dimethoxy-1,3-dihydro-2H-3-benzazepin-2-one with ethanol and then followed by addition of (7S)-[3,4-dimethoxybicyclo[4.2.0] octa-1,3,5-trien-7-yl]-N-methylmethan-amine hydro chloride with ethanol and water and final crystallization from toluene / 1-methyl-2-pyrrolidinone mixture affords α - crystalline form of Ivabradine hydrochloride.

US 2006/0194962 A1 discloses a process for preparation of the β-crystalline form of Ivabradine hydrochloride, which process is characterized in that a mixture of Ivabradine hydrochloride and water or a mixture of Ivabradine hydrochloride, isopropanol and water is heated until dissolution is complete and is then progressively cooled until crystallization is complete, and the crystals formed are collected.

US 2006/0194963 A1 also discloses a process for preparation of the y-crystalline form of Ivabradine hydrochloride, which process is characterized in that a mixture of Ivabradine hydrochloride and 2-ethoxyethanol, a mixture of Ivabradine hydrochloride, 2-ethoxyethanol and water, or a mixture of Ivabradine hydrochloride, ethanol and water is heated until dissolution is complete and is then cooled until crystallization is complete, and the product is collected by filtration.

US 2006/0194965 A1 discloses a process for the preparation of βd-crystalline form of Ivabradine hydrochloride, which process is characterized in that a mixture of Ivabradine hydrochloride and water or a mixture of Ivabradine hydrochloride, isopropanol and water is heated until dissolution is complete and is then progressively cooled until crystallization is complete and the crystals thereby formed are collected and dehydrated.

US 2006/01945964 A1 relates also to a process for preparation of the γd-crystalline form of Ivabradine hydrochloride, which process is characterized in that a mixture of Ivabradine hydrochloride and 2-ethoxyethanol, a mixture of Ivabradine hydrochloride, 2-ethoxyethanol and water, or a mixture of Ivabradine hydrochloride, ethanol and water is heated until dissolution is complete and is then cooled until crystallization is complete, and the crystals obtained are collected by filtration and dehydrated.

US 2007/0082886 discloses [delta]d-crystalline form of Ivabradine hydrochloride and a process for the preparation of the [delta]d-crystalline form of Ivabradine hydrochloride, wherein acetonitrile or a mixture of acetonitrile and water is preheated, Ivabradine hydrochloride is added, the solution obtained is allowed to cool at room temperature, held at room temperature until crystallisation is complete, and the crystals formed are dehydrated.

### OBJECT OF INVENTION:

An object of the present invention is to provide a process for preparing Ivabradine of formula (I).

Another object of the present invention is to provide a novel intermediate of formula (IV) for the preparation of Ivabradine hydrochloride.

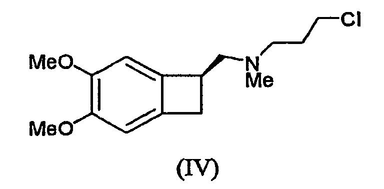

### SUMMARY OF THE INVENTION:

The present invention provides a process for preparing Ivabradine of formula (I) or its phamacetically acceptable salts, solvates or hydrates thereof via novel intermediate of formula (IV).

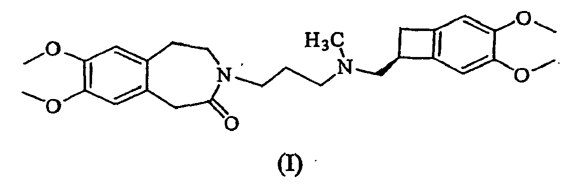

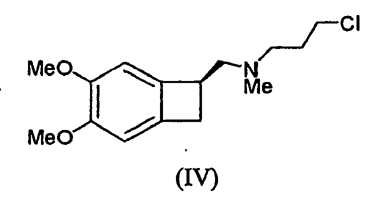

The present invention relates to novel intermediate of formula (IV) and its process for preparation.

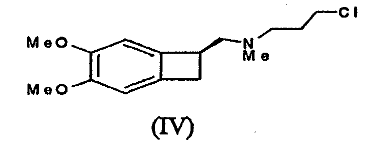

The present invention provides a novel intermediate for the preparation of Ivabradine hydrochloride with high yield and chiral purity.

### DETAILED DESCRIPTION OF THE INVENTION:

The present invention provides a process for preparing Ivabradine of formula (I) or its pharmaceutically acceptable salts, solvates, hydrate thereof, 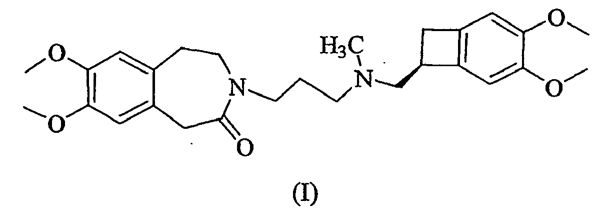 by reacting 3-chloro-N-{[(7S)-3,4-dimethoxybicyclo[4.2.0]octa-1,3,5-trien-7-yl]methyl}-N-methylpropan-I-amine of formula (IV), 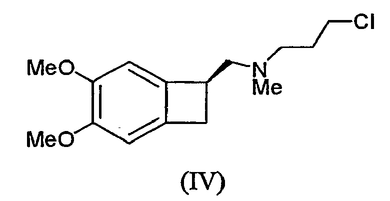 with 7,8-Dimethoxy-1,3,4,5-tetrahydro-benzo[d]azepin-2-one of formula (V) or its alkaline salt 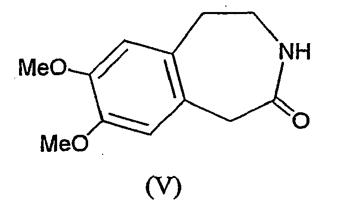 in presence of base in suitable solvent to provide Ivabradine of formula (I). This can be subsequently converted to its pharmaceutically acceptable salts, solvate or hydrate thereof.

The condensation reaction of compound of formula (IV) with compound of formula (V) is preferably carried out in presence of base selected from sodium hydroxide, potassium hydroxide, sodium carbonate, potassium carbonate, sodium methoxide, sodium ethoxide, potassium tert-butoxide, sodium hydride and the like. The preferred base is potassium carbonate or potassium tert-butoxide.

More preferably, compound of formula (V) is treated with alkali metal hydroxide, carbonate or alkoxide to form alkali metal salt of formula (V), which is further reacted with compounds for formula (IV) to provide Ivabradine.

The reaction is preferably carried out in suitable solvent. The solvent system is preferably selected so as to facilitate the condensation reaction and to allow subsequent separation of Ivabradine. Advantageously, both compound of formula (IV) and the compound of formula (V) are dissolvable, at least partly, in the solvent system, at least at elevated temperatures. In the process, a mixture, slurry, or solution of compound of formula (V) and a solvent may be contacted with a compound of formula (IV), or conversely, a mixture, slurry, or solution of compound of formula (IV) and a solvent may be contacted with compound of formula (V). In another embodiment, both partners may be combined with a solvent system prior to being contacted together, whereby the solvent system used for compound (IV) may be identical with or different from the solvent system used for the compound (V). The solvent system can be comprised of a single solvent or a mixture of solvents.

Suitable solvents include polar protic or aprotic solvent selected from a lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide, dimethyl acetamide, acetonitrile or mixtures thereof.

The temperature of contact of both the compounds in the solvent system is from ambient to the boiling point of the solvent system, with elevated temperatures, but generally less than the boiling point, being preferred.

In a preferred embodiment of the present invention, there is provide a process for preparing Ivabradine of formula (I) or its pharmaceutically acceptable salt, preferably hydrochloride comprising reaction of 3-chloro-N-{[(7S)-3,4-dimethoxybicyclo[4.2.0]octa-1,3,5-trien-7-yl]methyl}-N-methylpropan-1-amine of formula (IV) with 7,8-Dimethoxy-1,3,4,5-tetrahydro-benzo[d]azepin-2-one of formula (V) in presence of base selected from potassium tert-butoxide in dimethylsulfoxide to provide Ivabradine of formula (I).

The present invention also provides 3-chloro-N-{[(7S)-3,4-dimethoxybicyclo [4.2.0] octa-1,3,5-trien-7-yl]methyl}-N-methylpropan-1-amine of formula (IV), a novel intermediate useful for the preparation of Ivabradine.

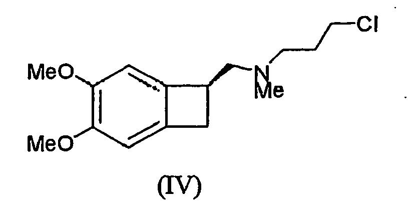

The present invention further provides a process for preparation of compound of formula (IV), which comprises reacting 1-[(7S)-3,4-dimethoxybicyclo[4.2.0]octa-1,3,5-trien-7-yl]-N-methylmethanamine of formula (VI) with 1-Bromo-3-chloropropane of formula (VII) in presence of base to form the novel compound of formula (IV)

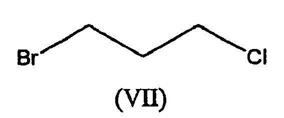

The reaction is preferably carried out in presence of base selected from sodium hydroxide, potassium hydroxide, sodium bicarbonate, and potassium bicarbonate. The preferred base potassium bicarbonate. The reaction can be preferably carried out in presence of solvent selected from lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide or dimethyl acetamide. Preferably, reaction is carried out in neat condition at room temperature. 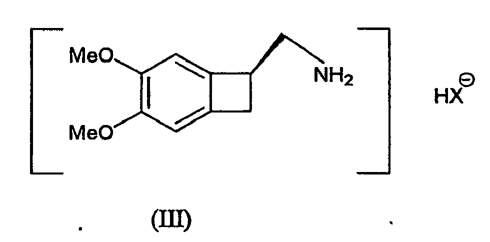 wherein HX = N-acetyl-L- gluatamic acid

(1S)-4,5-dimethoxy-1-(methylamine)-benzocyclobutane N-acetyl-L-gluatamic acid of formula (III) is further converted to 1-[(7S)-3,4-dimethoxybicyclo[4.2.0]octa-1,3,5-trien-7-yl]methanamine formula (VIII), which upon 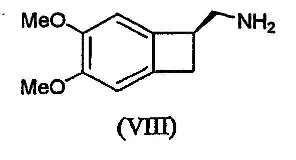 methylation gives 1-[(7S)-3,4-dimethoxybicyclo[4.2.0]octa-1,3;5-trien-7-yl]-N-methylmethanamine (VI) of formula

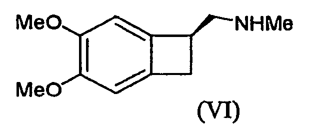

The reaction of Ivabradine base with HCl is preferably carried out in suitable solvent. The solvent system is preferably selected so as to facilitate the salt reaction and to allow subsequent separation of the resulting hydrochloride salt. Advantageously, both Ivabradine and the hydrochloric acid are dissolvable, at least partly, in the solvent system, at least at elevated temperatures. In the process, a mixture, slurry, or solution of Ivabradine and a solvent may be contacted with a hydrochloric acid, or conversely, a mixture, slurry, or solution of hydrochloric acid and a solvent may be contacted with Ivabradine. In another embodiment, both partners may be combined with a solvent system prior to being contacted together, whereby the solvent system used for hydrochloric acid may be identical with or different from the solvent system used for the Ivabradine. The solvent system can be comprised of a single solvent or a mixture of solvents. When two or more solvents are used, a two-phase reaction scheme may be used wherein the Ivabradine and hydrochloric acid are primarily reacted in one phase and the resulting Ivabradine hydrochloric acid compound is primarily present in the other phase due to, inter alia, solubility differences, etc. Suitable solvents include a lower alcohol (C₁- C₄) such as methanol, ethanol, isopropanol, n-propanol, n-butanol, iso-butanol, tert-butanol; ester such as ethyl acetate, isopropyl acetate, butyl acetate, iso-butyl acetate; ketone such as acetone, methyl ethyl ketone, methyl tert-butyl ketone; ether such as tetrahydrofuran, diethyl ether, Diisopropyl ether or dioxane.

The following examples describe the invention and are non-limiting in nature.

### BRIEF DETAILS OF DRAWINGS

The Figures are incorporated herein for reference only.
**Fig.I** is a characteristic X-ray powder diffraction pattern for Ivabradine Hydrochloride Form-I..
**Fig. II** is a characteristic differential scanning calorimetry thermogram of Ivabradine. Hydrochloride Form-I.
**Fig. III** is a characteristic X-ray powder diffraction pattern for crystalline form of (1S)-4,5-dimethoxy-1-(methylamine)-benzocyclobutane N-acetyl-L- gluatamic salt.
**Fig. IV** is a characteristic differential scanning calorimetry thermogram of crystalline form of (IS)-4,5-dimethoxy-1-(methylamine)-benzocyclobutane N-acetyl-L- gluatamic salt.
**Fig. V** is a characteristic thermal gravimetric analysis of crystalline form of (1S)-4,5-dimethoxy-1-(methylamine)-benzocyclobutane N-acetyl-L- gluatamic salt.
**Fig. VI** is a characteristic X-ray powder diffraction pattern for crystalline form of (1S)-4,5-dimetho3cy-1-(methylaminoniethyl)-benzocyclobutane-camphor sulphonate salt.
**Fig. VII** is a characteristic differential scanning calorimetry thermogram of crystalline form of (1S)-4,5-dimethoxy-1-(methylaminomethyl)-benzocyclobutane-camphor sulphonate salt.
**Fig**. **VIII** is a characteristic X-ray powder diffraction pattern for crystalline form of 7,8-dimethoxy-1,2,3,4-tetrahydro-2H-3-benzapin-2-one.
**Fig. IX** is a characteristic differential scanning calorimetry thermogram of crystalline form of 7,8-dimethoxy-1,2,3,4-tetrahydro-2H-3-benzapin-2-one.

### WORKING EXAMPLES:

### 1) Preparation of 3-(Chloro-propyl)-(3,4-dimetlioxy-bicyclo[4.2.0]octa-1(6),2,4-trien-7-ylmethyl)-methyl-amine

In a 1 liter Round bottom flask equipped with over head stirrer, thermometer pocket with thermometer and addition funnel with nitrogen gas inlet, (1S)-4,5-dimethoxy-1-(methyl aminomethyl)-benzocyclobutane (100 g, 0.48 mole), [compound is obtained after braking the camphorsulphonic acid salt of (1S)-4,5-dimethoxy-1-(methyl aminomethyl)-benzocyclo butane with sodium hydroxide], potassium carbonate (99.9 g, 0.72 mole) and 1-bromo-3-chloropropane (300 ml) was added and stirred for 5 Hr at room temperature. Further, MDC (300 ml) and water (300 ml) was added and stirred for 15 min. the layers were separated and MDC layer was washed with 5.0 % HCl (3 x 100 ml). To the aqueous layer pH was adjusted to pH 9-10 with liquor ammonia. Further, MDC (500 ml) was added and stirred for 15 min and layers were separated in similar manner as above. Finally, MDC layer was washed with water (2 x 100 ml) and dried over anhydrous sodium sulfate and solvent was distilled out to afford the title compound 3-(Chloro-propyl)-(3,4-dimethoxy-bicyclo[4.2.0]octa-1(6),2,4-trien-7-ylmethyl)-methyl-amine.

### 2) Preparation of Ivabradine Base

In a 1 liter Round bottom flask equipped with over head stirrer, thermometer pocket with thermometer and addition funnel with nitrogen gas inlet, 7,8-Dimethoxy-1,3,4,5-tetrahydro-benzo[d]azepin-2-one (81.86 g, 0.37 mole) was added followed by the addition of DMSO (160 ml) at room temperature. Further, Potassium-tert-butoxide (47.45 g, 0.42 mole) was added and a clear solution was observed and solid separate out within 5 min. The reaction mixture was stirred for 1 Hr and after stirring for 1 Hr, 3-(Chloro-propyl)-(3,4-dimethoxy-bicyclo[4.2.0]octa-1(6),2,4-trien-7-ylmethyl)-methyl-amine (110 g, 0.35 mole) was added being dissolved in DMSO (150 ml) within 1 Hr at 25-30°C. The reaction mixture was stirred for 4-5 Hrs at same temperature condition. After completion of the reaction the reaction mass was added to chilled water (1.5 L) and stirred for 15 min. After stirring the layers were separated and the organic layer was dried on sodium sulfate and solvent was completely distilled out to afford the title compound as Ivabradine base.

### 3) Preparation of Ivabradine Hydrochloride (Reference example)

In a 1 liter Round bottom flask equipped with over head stirrer, thermometer pocket with thermometer and addition funnel with nitrogen gas inlet, Ivabradine Base (100g, 0.21 mole) was added followed by the addition of acetonitrile (300 mL) at 20°C. Further, to the reaction mixture IPA HCl was added drop wise till the pH reaches to 1-2 and the solid was appeared. The reaction mass was cooled to 15-20°C and stirred for 12 Hr at 20°C. After stirring solid was formed and the solid was filtered and washed with acetonitrile under nitrogen atmosphere. Finally, the solid was recrystallized with acetonitrile under reflux condition for 2 Hrs and the solid was filtered and washed with acetonitrile to afford crystalline form of Ivabradine hydrochloride.

### 4) Preparation of Glutamte salt of (1S)-4,5-dimethoxy-1-(methytamine)-benzocyclobutane (Reference example)

In a 1 liter Round bottom flask equipped with over head stirrer, thermometer pocket with thermometer and addition funnel with nitrogen gas inlet (1S)- 4,5-dimethoxy-1-(methyl amine)-benzocyclobutane (100 g, 0.51 mole) and ethanol (2 L) was added. Further, N-acetyl glutamic acid (98.0 g, 0.51 mole) was added and reaction mass was stirred for 6 Hrs at 80°C. The reaction mass was cooled to 25-30°C and further cooled to 20°C and stirred for 8 Hrs and solid comes out. The solid mass was washed with ethanol (30 mL). The wet solid was crystallized with ethanol (800 mL). The reaction mass was cooled to 15-20°C and stirred for 8 Hrs and again the solid was washed with ethanol (40 mL) and dried in hot air oven for 5 Hrs at 60°C. The solid was again recrystallized with ethanol. To the solid mass water was added and further NaOH solution (230 mL) was added and stirred for 15 min. at 20°C. Further to the reaction mass MDC (370 mL) was added and the layers were separated out. MDC layer was washed with water and dried on sodium sulfate and MDC was distilled out completely under vacuum to afford the title compound as Glutamate salt of (1S)-4,5-dimethoxy-l-(methylaminomethyl)-benzocyclobutane.

### 5) Preparation of Camphor sulphonic acid salt of (1S)-4,5-dimethoxy-1-(methyl aminomethyl)-benzoeyelobutane (Reference example)

In a 1 liter Round bottom flask equipped with over head stirrer, thermometer pocket with thermometer and addition funnel with nitrogen gas inlet (1S)-4,5-dimethoxy-1-(methylaminomethyl)-benzocyclobutane (100 g, 0.48 mole) and (+) Camphor sulphonic acid (500 mL) and IPA (500, mL) was added and refluxed for 6 Hrs. The reaction mixture was slowly cooled to room temperature and stirred for 8 Hrs. After stirring the solid washed with IPA and dried in oven at 60°C for 6 Hrs. The solid was added into water (5 L) and basified with NaOH till the pH becomes 12. The reaction was stirred well and extracted with MDC. Finally the solid was dried on sodium sulfate and MDC was distilled out completely to afford Camphor sulphonic acid salt of (3,4-dimethoxy-bicyclo[4.2.0]octa-1(6),2,4-trien-7-ylmethyl)methylamine.

### 6) Preparation of crystalline salt of 7,8-Dimethoxy-1,3,4,5-tetrahydro-benzo[d]azepin-2-one (Reference example)

In a 1 liter Round bottom flask equipped with over head stirrer, thermometer pocket with thermometer and addition funnel with nitrogen gas inlet, 7,8-Dimethoxy-2-oxo-1,2,4,5-tetrahydro-benzo[d]azepine-3-carboxylic acid tert-butyl ester (100 g, 0.32 mole) and MDC (350 mL) was added. The reaction mixture was stirred for 15 min. at room temperature. Further the temperature was maintained upto 0-5°C. To the reaction mixture TFA (125 mL) was added drop wise within 2 Hrs at 0-5°C and stirred for 3 Hrs at 0-5°C. To the reaction mixture MDC (2 L) was added and the reaction mass was added into water (500 mL). The layers were separated and to the MDC layer sodium bicarbonate solution (620 mL) was added and again MDC layer was separated out and dried on sodium sulfate and solvent was distilled out completely. Finally to the residue ethyl acetate (250 mL) was added and stirred at 0-5°C for 4 Hrs. The solid was filtered at 0-5°C and finally washed with ethyl acetate to afford the crystalline form of 7,8-Dimethoxy-1,3,4,5-tetrahydro-benzo[d]azepin-2-one.

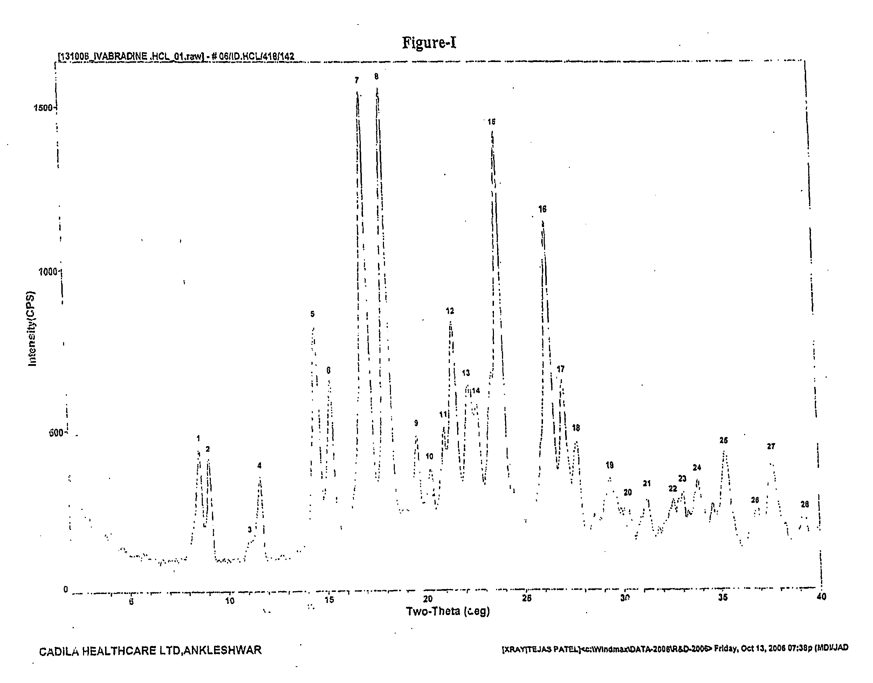

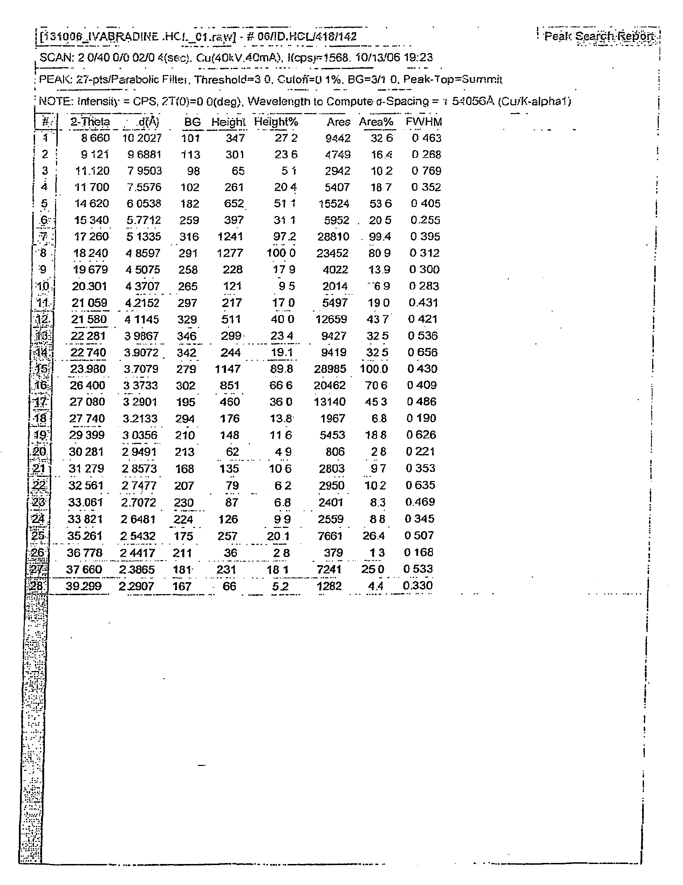

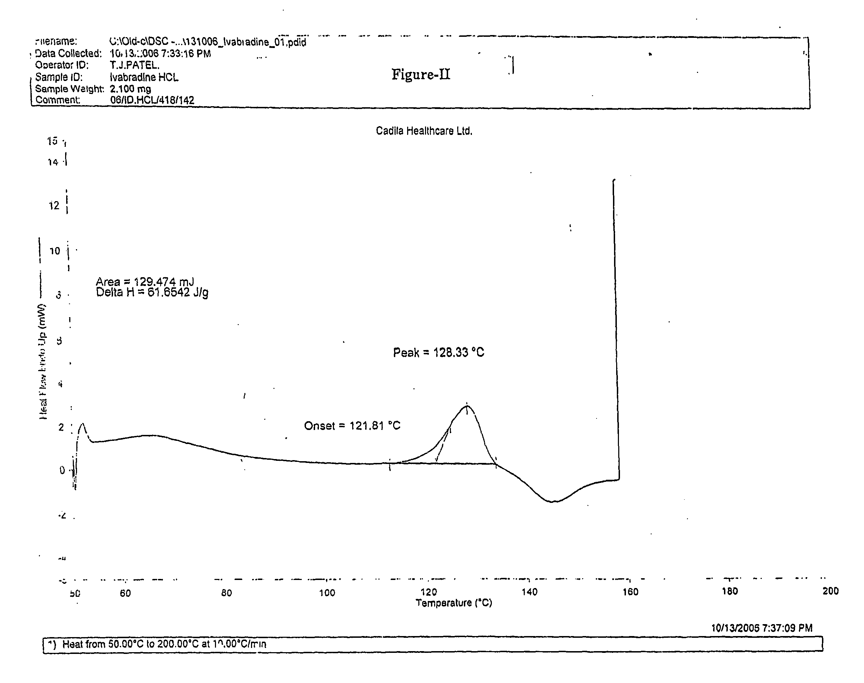

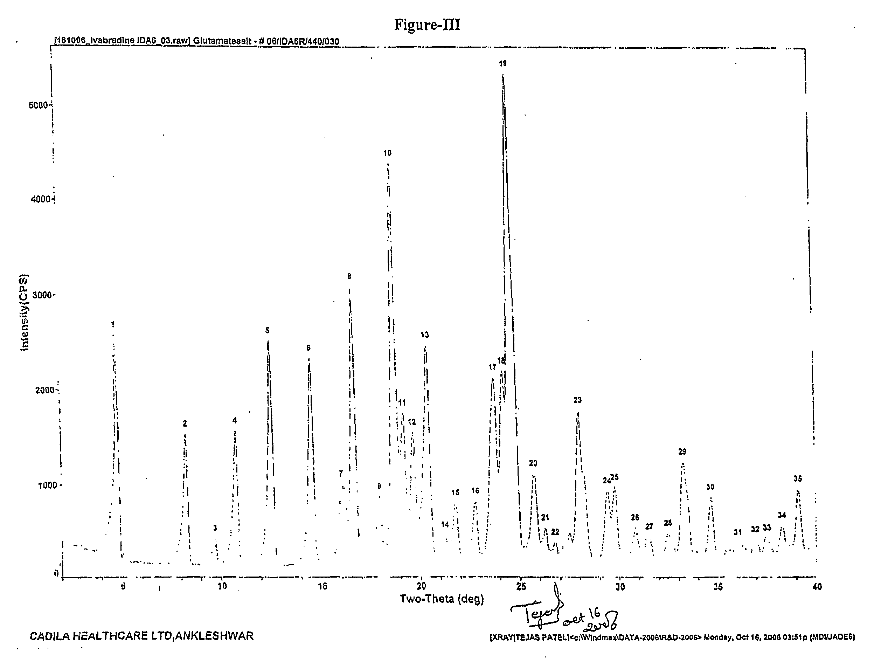

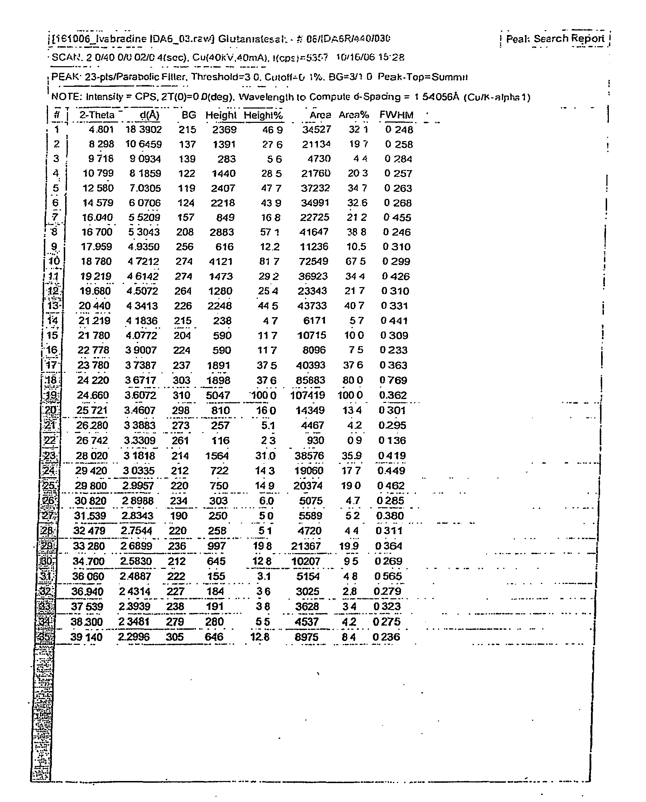

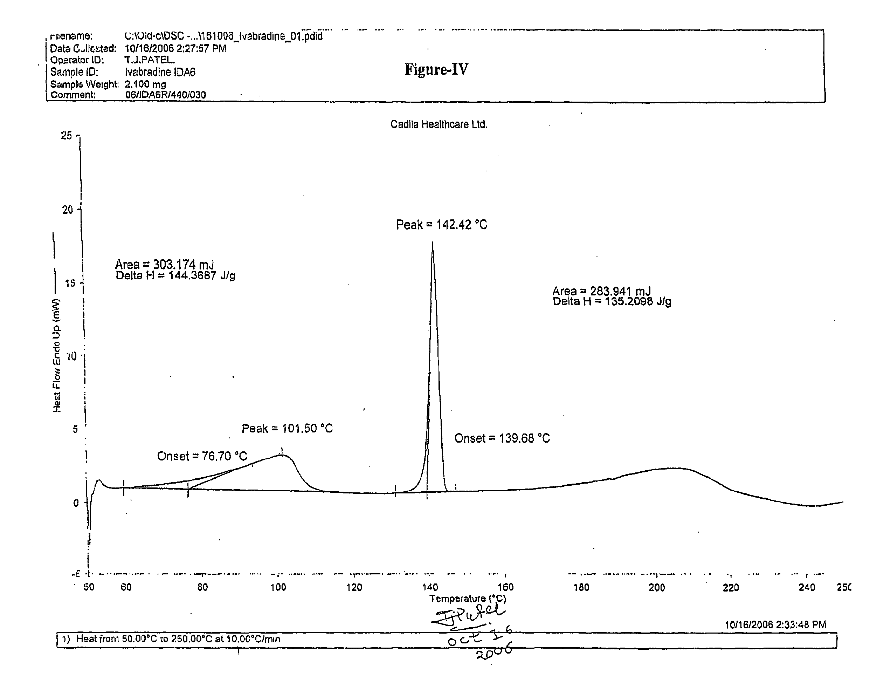

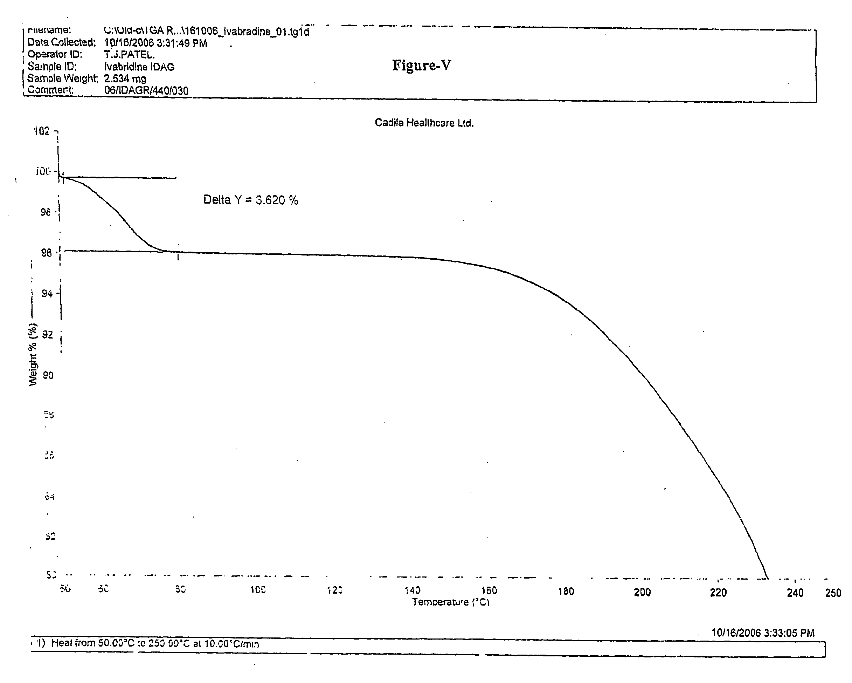

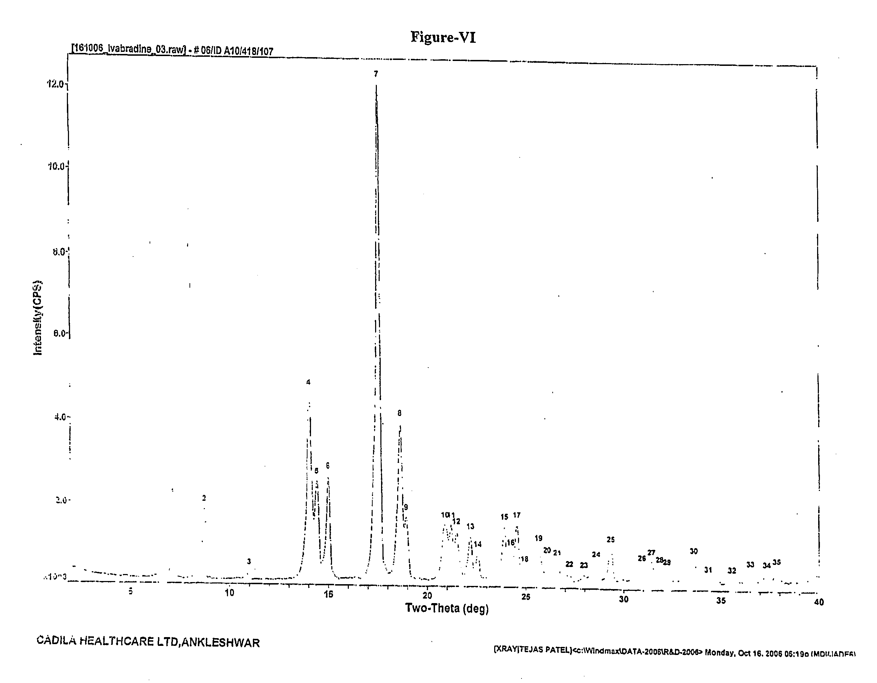

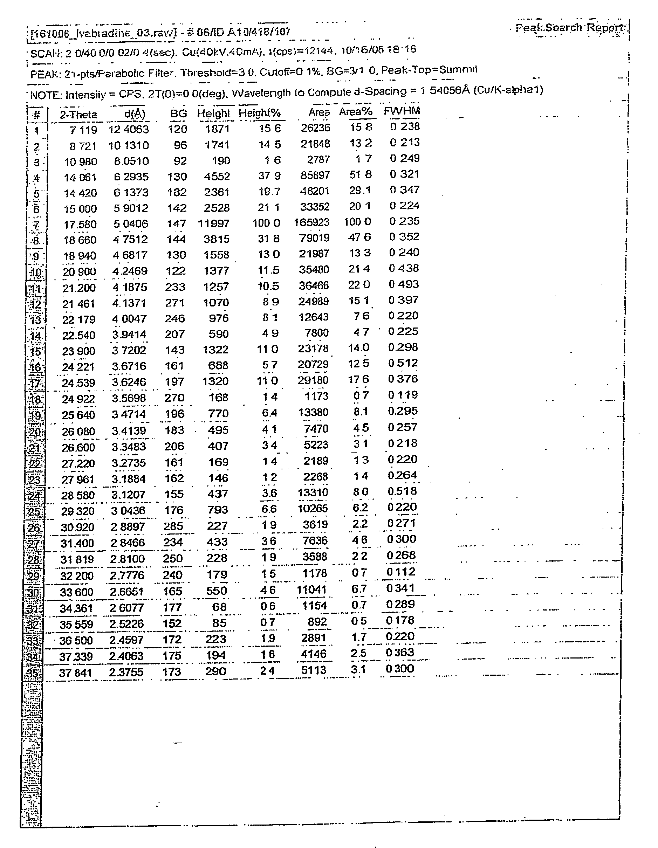

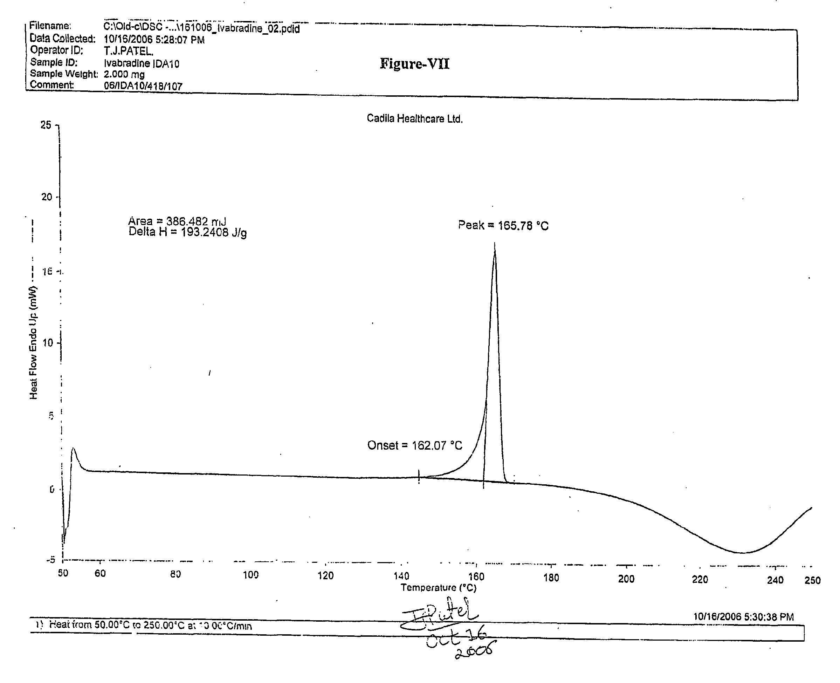

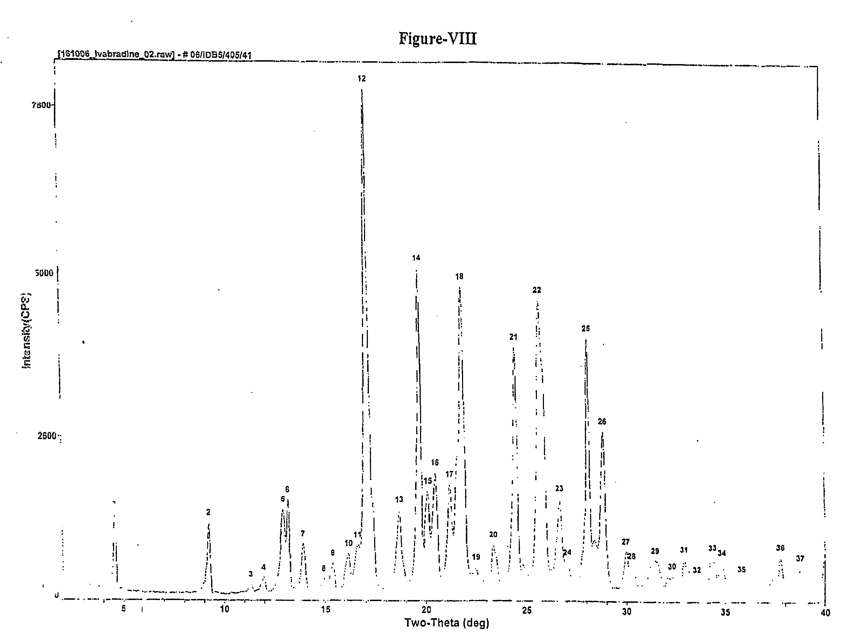

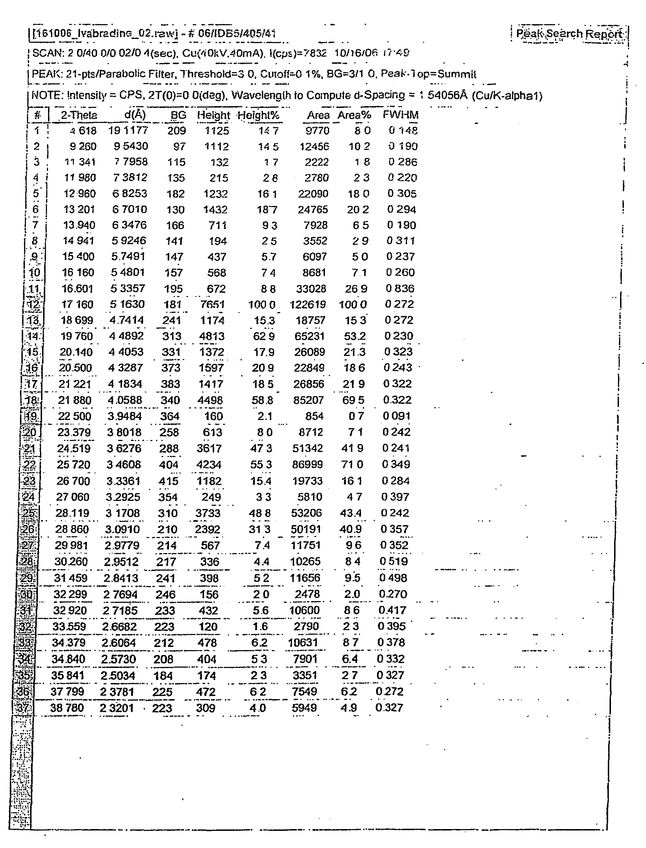

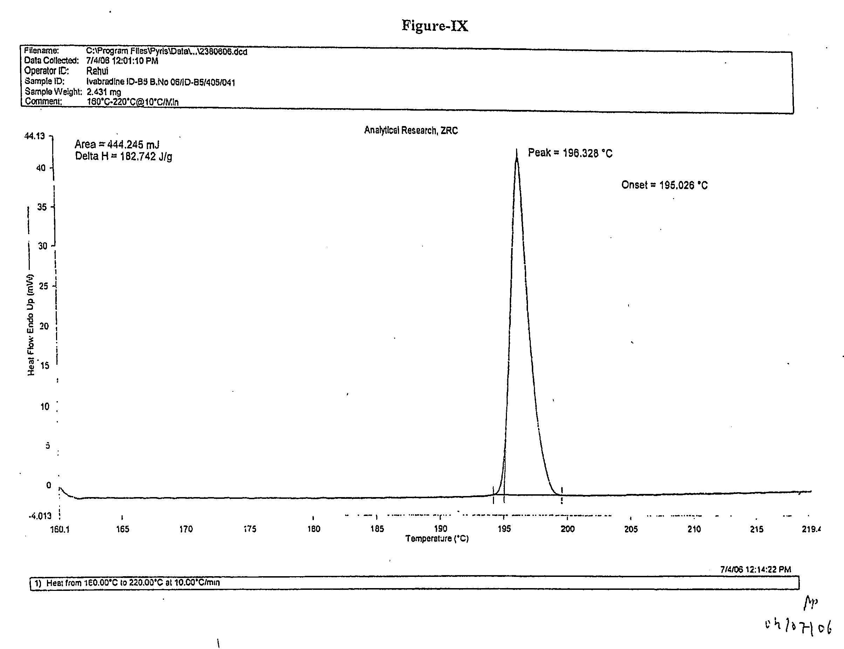

## Claims

1. A process for preparing Ivabradine of formula (I) or its pharmaceutically acceptable salts, solvates, hydrate thereof, 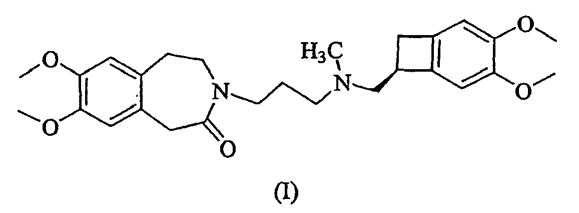 comprising reacting 3-chlo;o-N-{[(75)-3,4-dimethoxybicydo[4.2.0]octa-1,3,5-trien-7-yl]methyl}-N-methylpropan-1-amine of formula (IV), 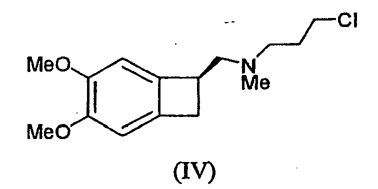 with 7,8-Dimethoxy-1,3,4,5-tetrahydro-benzo[d]azepin-2-one of formula (V) or its alkaline
salt 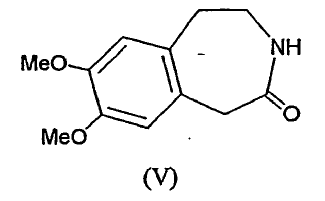 in presence of base in suitable solvent to provide Ivabradine of formula (I), and if desired, converting Ivabridine, of formula (I) to its pharmaceutically acceptable salts, solvate or hydrate form.

2. A process as claimed in claim 1, wherein said base is selected from sodium hydroxide, potassium hydroxide, sodium carbonate, potassium carbonate, sodium methoxide, sodium ethoxide, potassium tert-butoxide, and sodium hydride.

3. A process as claimed in- claim 2, wherein the base is potassium carbonate or potassium tert-butoxide.

4. A process as claimed in claim 1, wherein suitable solvent is selected from polar protic or aprotic solvents, selected from a lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide, dimethyl acetamide, acetonitrile or mixtures thereof

5. A process as claimed in claim 4, wherein said solvent is dimethyl sulfoxide.

6. A process for preparing Ivabradine of formula (I) or its pharmaceutically acceptable salt, preferably hydrochloride, comprising reacting 3-chloro-N-([(7S)-3,4-dimethoxybicyclo [4.2.0]octa-1,3,5-trien-7-yl]methyl}-N-methylpropan-1-amine of formula (IV) with 7,8-Dimethoxy-1,3,4,5-tetrahydro-benzo[d]azepin-2-one of formula (V) in presence of base selected from potassium tert-butoxide in dimethylsulfoxide to provide Ivabradine of formula (I).

7. 3-chloro-N-{[(7S)-3,4-dimethoxybicyclo[4.2.0]octa-1,3,5-trien-7-yl]methyl}-N-methylpropan-1-amine of formula (IV),.

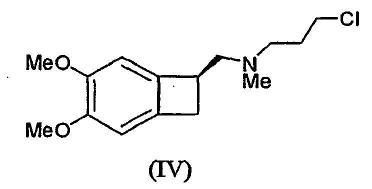

8. A process for preparing 3-chloro-N-{[(75)-3,4-dimethoxybicydo[4.2.0]octa-1,3,5-trien-7-yl]methyl}-N-methylpropan-1-amine of formula (IV), 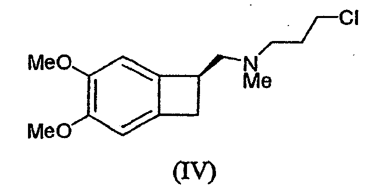 which comprises reacting 1-[(7S)-3,4-dimethoxybicyclo[4.2.0]octa-1,3,5-trien-7-yl]-N-methylmethanamine of formula (VI) with 1-Bromo-3-chloropropane of formula (VII) 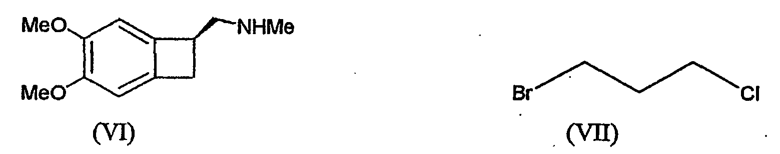 in presence of base to provide 3-chloro-N-{[(75)-3,4-dimethoxybicyclo[4.2.0]octa-1,3,5-trien-7-yl]methyl}-N-methylpropan-1-amine of formula (IV).

9. A process as claimed in claim 8, wherein base is selected from sodium hydroxide, potassium hydroxide, and sodium bicarbonate, preferably potassium bicarbonate.

10. A process as claimed in claim 8, wherein said reaction is carried out in solvent selected from lower alcohol (C₁- C₆) such as methanol, ethanol, isopropanol, n-propanol, n-butanol, iso-butanol, tert-butanol; ether such as tetrahydrofuran, diethyl ether, diisopropyl ether, dioxane; sulfoxide such as sulfolane or dimethyl sulfoxide, amides such as dimethyl formamide, dimethyl acetamide.

11. A process as claimed in claim 8, wherein said reaction is carried out in neat condition at room temperature.

12. A process as claimed in 8, wherein said 1-[(7S)-3,4-dimethoxybicyclo[4.2.0]octa-l,3,5-trien-7-yl]-N-methylmethanamine (VI) is prepared by converting (1S)-4,5-dimethoxy-1-(methylamine)-benzocyclobutane N-acetyl-L- gluatamic acid of formula (III) to 1-[(7S)-3,4-5 dimethoxybicyclo[4.2.0]octa-1,3,5-trien-7-yl]methanamine formula (VIII),which upon 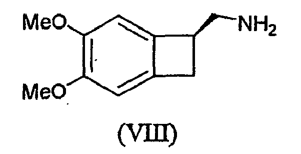 methylation gives 1-[(7S)-3,4-dimethoxybicyclo[4.2.0]octa-1,3,5-trien-7-yl]-N-methylmethanamine (VI) of formula

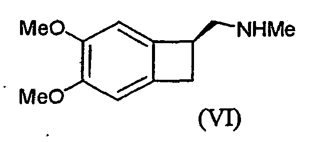

## Patentansprüche

1. Verfahren zur Herstellung von Ivabradin der Formel (I) oder pharmazeutisch unbedenklicher Salze, Solvate, pharmazeutisch unbedenklichen Hydrats davon 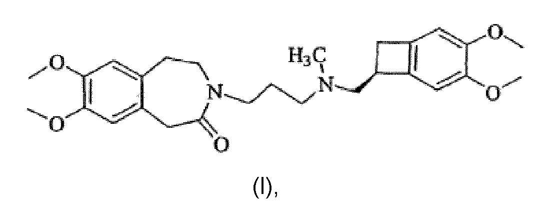 umfassend das Verreagieren von 3-Chlor-N-{[(7S)-3,4-dimethoxybicyclo[4.2.0]octa-1,3,5-trien-7-yl]methyl}-N-methylpropan-1-amin der Formel (IV) 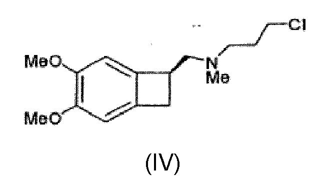 mit 7,8-Dimethoxy-1,3,4,5-tetrahydro-benzo[d]azepin-2-on der Formel (V) oder seines Alkalisalzes 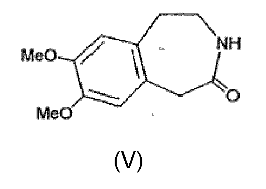 in Gegenwart einer Base in geeignetem Lösemittel, um Ivabradin der Formel (I) bereitzustellen und, falls gewünscht, das Umwandeln von Ivabradin der Formel (I) in seine pharmazeutisch unbedenklichen Salze, Solvate oder seine pharmazeutisch unbedenkliche Hydratform.

2. Verfahren nach Anspruch 1, wobei die Base aus Natriumhydroxid, Kaliumhydroxid, Natriumcarbonat, Kaliumcarbonat, Natriummethoxid, Natriumethoxid, Kalium-tert-butoxid und Natriumhydrid ausgewählt wird.

3. Verfahren nach Anspruch 2, wobei die Base Kaliumcarbonat oder Kalium-tert-butoxid ist.

4. Verfahren nach Anspruch 1, wobei das geeignete Lösemittel ausgewählt wird aus polaren protischen oder aprotischen Lösemitteln, die ausgewählt werden aus einem geringwertigen Alkohol (C₁-C₆) wie Methanol, Ethanol, Isopropanol, n-Propanol, n-Butanol, iso-Butanol, tert-Butanol, aus Ether wie Tetrahydrofuran, Diethylether, Diisopropylether, Dioxan, aus Sulfoxid wie Sulfolan oder Dimethylsulfoxid, aus Amiden, wie Dimethylformamid, Dimethylacetamid, Acetonitril oder Mischungen daraus.

5. Verfahren nach Anspruch 4, wobei das Lösemittel Dimethylsulfoxid ist.

6. Verfahren zur Herstellung von Ivabradin der Formel (I) oder seines pharmazeutisch unbedenklichen Salzes, vorzugsweise Hydrochlorid, umfassend das Verreagieren von 3-Chlor-N-{[(7S)-3,4-dimethoxybicyclo[4.2.0]octa-1,3,5-trien-7-yl]methyl]-N-methylpropan-1-amin der Formel (IV) mit 7,8-Dimethoxy-1,3,4,5-tetrahydro-benzo[d]azepin-2-on der Formel (V) in Gegenwart einer Base, die aus Kalium-tert-butoxid ausgewählt ist, in Dimethylsulfoxid, um Ivabradin der Formel (I) bereitzustellen.

7. 3-Chlor-N-{[(7S)-3,4-dimethoxybicyclo[4.2.0]octa-1,3,5-trien-7-yl]methyl}-N-methylpropan-1-amin der Formel (IV)

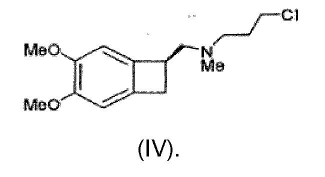

8. Verfahren zur Herstellung von 3-Chlor-N-{[(7S)-3,4-dimethoxybicyclo[4.2.0]octa-1,3,5-trien-7-yl]methyl}-N-methylpropan-1-amin der Formel (IV) 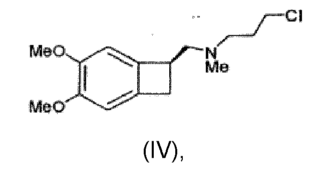 umfassend das Verreagieren von 1-[(7S)-3,4-Dimethoxybicyclo[4.2.0]octa-1,3,5-trien-7-yl]-N-methylmethanamin der Formel (VI) mit 1-Brom-3-chlorpropan der Formel (VII) 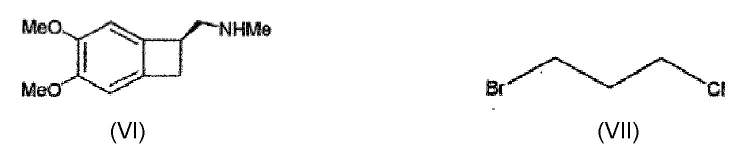 in Gegenwart einer Base, um 3-Chlor-N-{[(7S)-3,4-dimethoxybicyclo[4.2.0]octa-1,3,5-trien-7-yl]methyl}-N-methylpropan-1-amin der Formel (IV) bereitzustellen.

9. Verfahren nach Anspruch 8, wobei die Base ausgewählt wird aus Natriumhydroxid, Kaliumhydroxid und Natriumbicarbonat, vorzugsweise Kaliumbicarbonat.

10. Verfahren nach Anspruch 8, wobei die Reaktion in einem Lösemittel durchgeführt wird, das ausgewählt wird aus geringwertigem Alkohol (C₁ - C₆) wie Methanol, Ethanol, Isopropanol, n-Propanol, n-Butanol, iso-Butanol, tert-Butanol, aus Ether wie Tetrahydrofuran, Diethylether, Diisopropylether, Dioxan, aus Sulfoxid wie Sulfolan oder Dimethylsulfoxid, aus Amiden wie Dimethylformamid, Dimethylacetamid.

11. Verfahren nach Anspruch 8, wobei die Reaktion in unverdünntem Zustand bei Raumtemperatur durchgeführt wird.

12. Verfahren nach Anspruch 8, wobei das 1-[(7S)-3,4-Dimethoxybicyclo[4.2.0]octa-1,3,5-trien-7-yl]-N-methylmethanamin (VI) hergestellt wird, indem (1S)-4,5-Dimethoxy-1-(methylamin)-benzocyclobutan-N-acetyl-L-glutaminsäure der Formel (III) in 1-[(7S)-3,4-Dimethoxybicyclo[4.2.0]octa-1,3,5-trien-7-yl]methanamin der Formel (VIII) umgewandelt wird 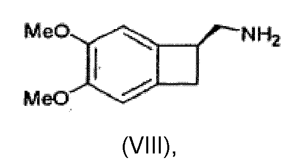 das nach der Methylierung 1-[(7S)-3,4-Dimethoxybicyclo[4.2.0]octa-1,3,5-trien-7-yl]-N-methylmethanamin der Formel (VI) ergibt

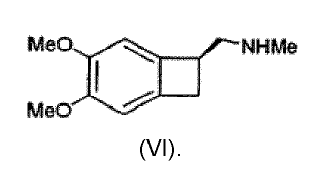

## Revendications

1. Processus de préparation de l'ivabradine de formule (I) ou de sels, solvates et hydrates pharmaceutiquement acceptables de celle-ci, 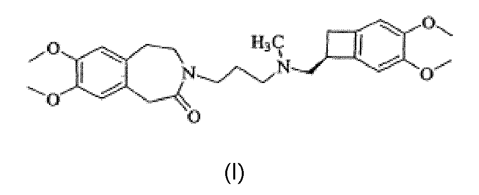 comprenant la réaction entre la 3-chloro-N-{[(7S)-3,4-diméthoxybicyclo[4.2.0]octa-1,3,5-trién-7-yl]méthyl}-N-méthylpropan-1-amine de formule (IV), 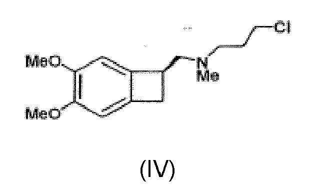 et la 7,8-diméthoxy-1,3,4,5-tétrahydro-benzo[d]azépin-2-one de formule (V) ou un sel alcalin de celle-ci 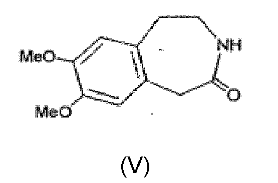 en présence d'une base dans un solvant approprié pour obtenir l'ivabradine de formule (I), et si désiré, convertir l'ivabradine de formule (I) en ses sels ou formes solvatées ou hydratées pharmaceutiquement acceptables.

2. Procédé selon la revendication 1, où ladite base est sélectionnée parmi les suivantes : hydroxyde de sodium, hydroxyde de potassium, carbonate de sodium, carbonate de potassium, méthoxyde de sodium, éthoxyde de sodium, tert-butoxyde de potassium et hydrure de sodium.

3. Procédé selon la revendication 2, où la base est le carbonate de potassium ou le tert-butoxyde de potassium.

4. Procédé selon la revendication 1, où le solvant approprié est sélectionné parmi des solvants polaires protiques ou aprotiques choisis parmi un alcool inférieur en (C₁-C₆) comme le méthanol, l'éthanol, l'isopropanol, le n-propanol, le n-butanol, l'iso-butanol ou le tert-butanol ; un éther comme le tétrahydrofurane, l'éther diéthylique, l'éther diisopropylique ou le dioxane ; un sulfoxyde comme le sulfolane ou sulfoxyde diméthylique, des amides comme le diméthylformamide, le diméthylacétamide, l'acétonitrile ou des mélanges de ceux-ci.

5. Procédé selon la revendication 4, où ledit solvant est le sulfoxyde diméthylique.

6. Procédé de préparation de l'ivabradine de formule (I) ou d'un sel pharmaceutiquement acceptable de celle-ci, préférablement un hydrochlorure, comprenant la réaction entre la 3-chloro-N-{[(7S)-3,4-diméthoxybicyclo[4.2.0]octa-1,3,5-trién-7-yl]méthyl}-N-méthylpropan-1-amine de formule (IV) et la 7,8-diméthoxy-1,3,4,5-tétrahydro-benzo[d]azépin-2-one de formule (V) en présence d'une base sélectionnée parmi le tert-butoxyde de potassium dans le sulfoxyde de diméthyle pour obtenir l'ivabradine de formule (I).

7. 3-Chloro-N-([(75)-3,4-diméthoxybicydo[4.2.0]octa-1,3,5-trién-7-yl]méthyl)-N-méthylpropan-1-amine de formule (IV),

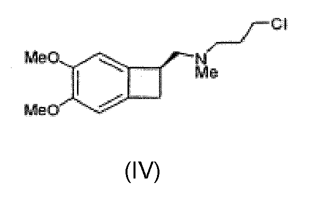

8. Procédé de préparation de la 3-chloro-N-{[(7S)-3,4-diméthoxybicyclo[4.2.0]octa-1,3,5-trién-7-yl]méthyl}-N-méthylpropan-1-amine de formule (IV), 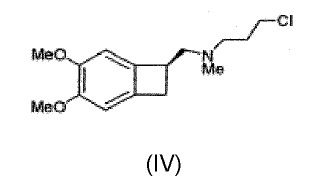 qui comprend la réaction entre la 1-[(7S)-3,4-diméthoxybicyclo[4.2.0]octa-1,3,5-trién-7-yl]-N-méthylméthanamine de formule (VI) et le 1-bromo-3-chloropropane de formule (VII) 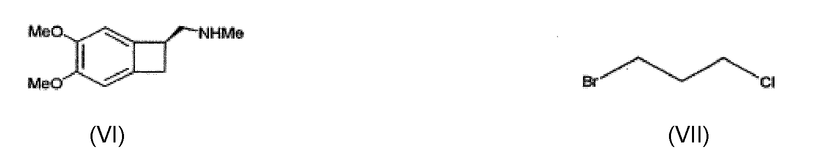 en présence d'une base pour obtenir la 3-chloro-N-{[(7S)-3,4-diméthoxybicyclo[4.2.0]octa-1,3,5-trién-7-yl]méthyl}-N-méthylpropan-1-amine de formule (IV).

9. Procédé selon la revendication 8, où ladite base est sélectionnée parmi l'hydroxyde de sodium, l'hydroxyde de potassium et le bicarbonate de sodium, préférablement le bicarbonate de potassium.

10. Procédé selon la revendication 8, où ladite réaction est effectuée dans un solvant sélectionné parmi un alcool inférieur en (C₁-C₆) comme le méthanol, l'éthanol, l'isopropanol, le n-propanol, le n-butanol, l'iso-butanol ou le tert-butanol ; un éther comme le tétrahydrofurane, l'éther diéthylique, l'éther diisopropylique ou le dioxane ; un sulfoxyde comme le sulfolane ou sulfoxyde diméthylique, des amides comme le diméthylformamide ou le diméthylacétamide.

11. Procédé selon la revendication 8, où ladite réaction est effectuée en l'absence d'un solvant à température ambiante.

12. Procédé selon la revendication 8, où ladite 1-[(7S)-3,4-diméthoxybicyclo[4.2.0]octa-1,3,5-trién-7-yl]-N-méthylméthanamine (VI) est préparée par conversion de l'acide (1S)-4,5-diméthoxy-1-(méthylamine)-benzocyclobutane-N-acétyl-L-glutamique de formule (III) en la 1-[(7S)-3,4-diméthoxybicyclo[4.2.0]octa-1,3,5-trién-7-yl]méthanamine de formule (VIII), 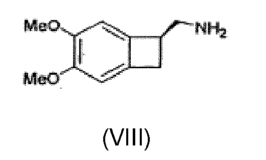 qui, après méthylation, donne la 1-[(7S)-3,4-diméthoxybicyclo[4.2.0]octa-1,3,5-trién-7-yl]-N-méthylméthanamine (VI) de formule
